# EUROPEAN PATENT SPECIFICATION

(11) **EP 4 309 492 B1**
(45) Date of publication and mention of the grant of the patent: **24.09.2025**
(21) Application number: 23184759.1
(22) Date of filing: 11.07.2023
(51) Int. Cl.: A01G 9/12, G01N 33/00

(54) **MEASUREMENT MODULE**
MESSMODUL
MODULE DE MESURE

(30) Priority: 19.07.2022 JP 2022114790
(43) Date of publication of application: 24.01.2024
(73) Proprietor: Sintokogio, Ltd., Nagoya-shi, Aichi 450-6424 (JP)
(72) Inventor: MIZUTANI, Manase, Nagoya-shi, Aichi, 450-6424 (JP)
(74) Representative: Hoffmann Eitle

(56) References cited:
- WO-A1-2018/095850
- JP-A- 2002 065 822

## Description

### TECHNICAL FIELD

The present disclosure relates to a measurement module.

### BACKGROUND

Japanese Patent Application Publication No. 2020-95034 discloses a device for non-destructively measuring a metabolic product of a plant. The device measures a metabolic product of a plant by irradiating a part of a flower stem or a petiole of the plant with light and measuring a light amount difference between the transmitted light or the reflected light and the irradiated light. In the case of measuring an upwardly extending plant, the device is fixed to at least one of the petiole and the peduncle, which is the passage of the metabolic product of the plant, and is supported by a strut.

JP 2002 065822 A discloses an artificial plant comprising ventilation means and having a photocatalytic function. WO2018/095850 A1 discloses a horticulture arrangement comprising a lighting system.

### SUMMARY

The device described in Japanese Patent Application Publication No. 2020-95034 can measure only specific parts of a plant such as leaf bodies and fruits. Therefore, the device described in Japanese Patent Application Publication No. 2020-95034 may not be able to continuously measure the state of a plant having a height of several meters, such as tomato. The present invention provides a measurement module capable of continuously detecting the state of a plant with a simple configuration even if the plant has a high height.

A measurement module according to the present invention includes a hollow strut, at least one introduction port, at least one odor sensor, and a pump. The struts are erected to support the plant. The at least one introduction port communicates the interior of the strut with the external. The at least one odor sensor is disposed at a position corresponding to the at least one introduction port in the interior of the strut to detect the odor of the plant. The pump is connected to the strut and draws outside air from the introduction port into the interior of the strut.

According to the present invention, it is possible to provide a measurement module capable of continuously detecting the state of a plant with a simple configuration even if the plant has a high height.

### BRIEF DESCRIPTION OF THE DRAWINGS

FIG. 1 is a configuration diagram schematically illustrating an example of a measurement module according to an embodiment.
FIG. 2 is a diagram schematically showing the interior of the measurement module shown in FIG. 1.
FIG. 3 is a diagram schematically illustrating an interior of a measurement module according to a modification.

### DETAILED DESCRIPTION

### [SUMMARY OF EMBODIMENTS OF THE PRESENT DISCLOSURE]

First, an outline of an embodiment of the present disclosure will be described.

The invention is achieved with the appended claims. A measurement module according to one side surface of the present disclosure includes a hollow strut, at least one introduction port, at least one odor sensor, and a pump. The struts are erected to support the plant. The at least one introduction port communicates the interior of the strut with the external. The at least one odor sensor is disposed at a position corresponding to the at least one introduction port in the interior of the strut to detect the odor of the plant. The pump is connected to the strut and draws outside air from the introduction port into the interior of the strut.

In this measurement module, a hollow strut is erected to support the plant. In the interior of the strut, an odor sensor that detects the odor of the plant is disposed at a position corresponding to at least one introduction port that communicates the interior of the strut with the external. A pump connected to the strut draws outside air from the at least one introduction port into the interior of the strut. The plant is supported by the strut, and odor emitted from the plant is taken into the interior of the strut through at least one introduction port by suction of the pump, and is detected by at least one odor sensor. The odor emitted from the plant varies depending on the state of the plant. Since the measurement module detects the odor by the odor sensor, it is not necessary to perform advance preparation such as determining the measurement site in detail and applying light to the measurement site as compared with a case where the state of the plant is detected using light. Therefore, the measurement module can continuously detect the state of the plant even if the position of the measurement site is shifted due to growth of the plant or application of an external force such as wind. The measurement module can be configured with a smaller number of components compared to a case where piping and struts are separately provided by using a flow path that guides an odor to an odor sensor also as a strut that supports a plant. Therefore, according to this measurement module, even when the height of the plant is high, the state of the plant can be continuously detected with a simple configuration.

In a further embodiment of the measurement module, the strut may have a hollow columnar main body portion and at least one hollow box-shaped housing portion communicating with an interior of the main body portion, the at least one introduction port may communicate between the at least one housing portion and the external, and the at least one odor sensor may be disposed in the interior of the at least one housing portion. In this case, the measurement module may incorporate odorant into the housing portion and increase the concentration of odorant in the atmosphere in the interior of the housing portion. Therefore, the measurement module can more accurately detect the state of the plant.

In a further embodiment of the measurement module, a connection portion between each housing portion and the main body portion may be smaller in diameter than each housing portion. In this case, the measurement module can easily entangle the plant itself and a cord or the like for fixing the plant to the strut with the connection portion.

In a further embodiment of the measurement module, the at least one housing portion is removably attached to the main body portion. In this case, the measure can arrange the odor sensor at a desired position such as a position where the leaf body of the plant is attached.

In a further embodiment of the measurement module, the strut may have a hollow columnar shape, and the at least one introduction port may be provided on a side surface of the strut. In this case, it is not necessary to provide a space for arranging the odor sensor in the post external. Therefore, this measurement module can achieve space saving.

In a further embodiment of the measurement module, the introduction port may include a tapered surface that diverges from an interior of the strut toward an exterior of the strut. In this case, the measurement module may take more air from the periphery of the plant into the interior of the strut.

In a further embodiment, the measurement module may further include an environmental sensor configured to detect at least one of a temperature, humidity, and carbon dioxide concentration. In this case, the measurement module may detect the state of the plant based on the detection result of the environmental sensor.

In a further embodiment of the measurement module, the at least one introduction port may include a plurality of introduction ports, the at least one odor sensor may include a plurality of odor sensors, and each of the plurality of odor sensors may be disposed at a position corresponding to each of the plurality of introduction ports. In this case, since the measurement module can detect odors at a plurality of locations in the plant, the state of the plant can be detected more accurately.

In a further embodiment of the measurement module, the plurality of introduction ports and the plurality of odor sensors may be arranged at equal intervals in a height direction of the strut. In this case, since the measurement module can detect odors at a plurality of locations from the lower side to the upper side in the plant, the measurement module can more accurately detect the state of the plant even if the height of the plant is high.

### [Examples of Embodiments of the Present Disclosure]

Hereinafter, embodiments of the present disclosure will be described in detail with reference to the drawings. In the description of the drawings, the same elements are denoted by the same reference numerals, and redundant description is omitted. The dimensional ratios in the figures are not necessarily consistent with those in the description.

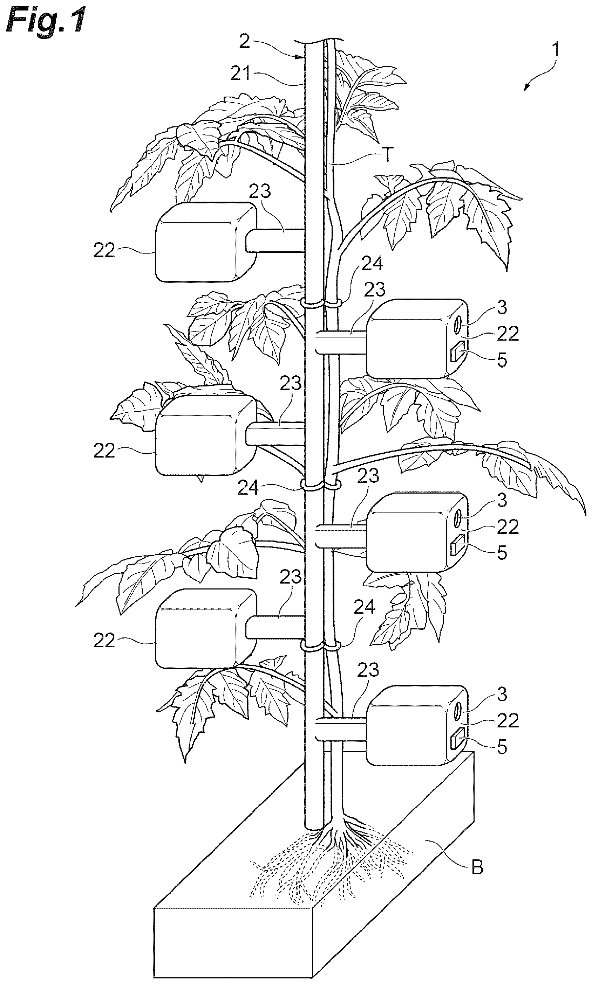

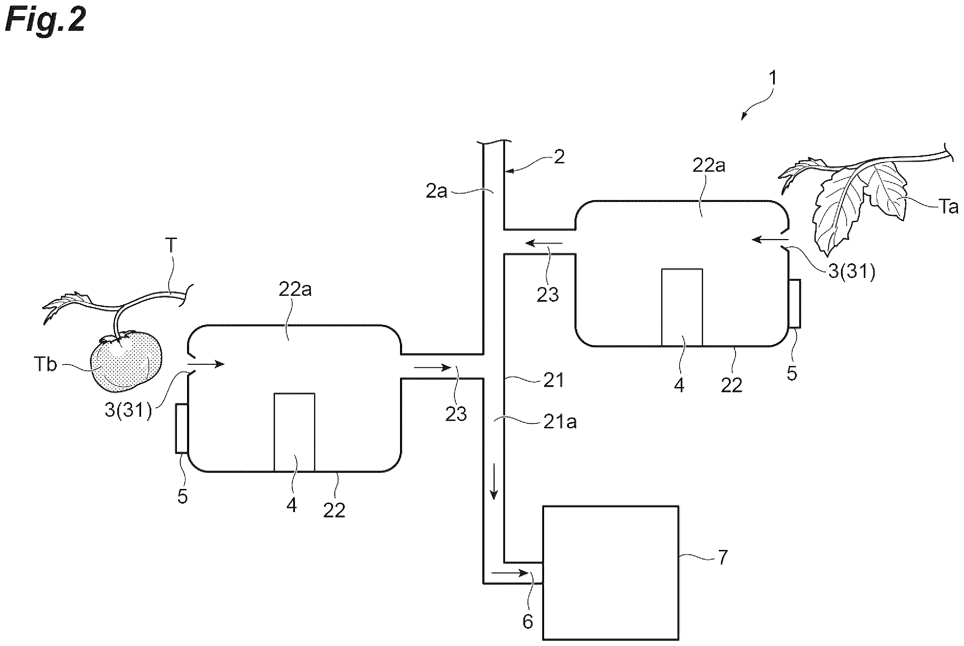

[Example of Measurement Module] FIG. 1 is a configuration diagram schematically illustrating an example of a measurement module 1 according to an embodiment. FIG. 2 is a schematic view of the interior of the measurement module 1 shown in FIG. 1. The measurement module 1 shown in FIGS. 1 and 2 is a module for measuring the state of a plant T while supporting the plant T. Examples of the plant T states include the plant T health state and the plant T growth state. The plant T is a plant serving as a measurement target and is, for example, tomato, apple, or the like. As an example, the plant T is cultivated in a box-shaped a culture medium B. In FIG. 2, illustration of the culture medium B is omitted.

The measurement module 1 is configured to communicate with external servers (not shown) by wireless or wired communication, and is configured to be able to transmit various types of data such as measured information to the servers. The server may be configured as a computer system including a processor such as a central processing unit (CPU), memories such as a random-access memory (RAM) and a read only memory (ROM), input/output devices such as a touch panel, a mouse, a keyboard, and a display, and a communication device such as a network card. The servers realize the function of recognizing the state of the plant T by operating the respective hardware under the control of the processor based on the computer program stored in the memory.

The measurement module 1 includes a strut 2, an introduction port 3, an odor sensor 4, an environmental sensor 5, a gas pipe 6, and a pump 7. The strut 2 is hollow and stands on the culture medium B to support the plant T. The strut 2 has a main body portion 21 and multiple a housing portion 22.

The main body portion 21 is a standing portion of the strut 2. The main body portion 21 is placed, for example, near the stalk of the plant T. The main body portion 21 may stand on the culture medium B, or may stand adjacent to the culture medium B when the culture medium B is small or when damage to the culture medium B is to be prevented. As shown in FIG. 2, the main body portion 21 has a hollow columnar shape. An interior 21a of the main body portion 21 is a cavity extending in the height direction of the main body portion 21. When the measurement module 1 is provided in a facility such as a vinyl house, the upper portion of the main body portion 21 may be tied to a cord 24 for fixing in the plant T extending in the horizontal direction with the ground above the plant T.

The plurality of the housing portion 22 are disposed outside the main body portion 21 and communicate with the interior 21a of the main body portion 21. Each the housing portion 22 is a portion of the strut 2 that contains the odor sensor 4. Each the housing portion 22 has the shape of a hollow box. An interior 22a of each such the housing portion 22 includes a space for housing each the odor sensor 4. Each the housing portion 22 has, for example, a size that can be handled by hand. As shown in FIG. 2, the housing portion 22 portion of each corner is a chamfered shape. Each the housing portion 22 may be integrally configured with the main body portion 21 and may be removably attached to the main body portion 21.

The strut 2 further includes a plurality of a connection portion 23. Each the connection portion 23 is placed between each the housing portion 22 and the main body portion 21 and connects each the housing portion 22 and the main body portion 21. Each the housing portion 22 communicates with the interior 21a of the main body portion 21 via each the connection portion 23. Each the connection portion 23 is made smaller in diameter than each the housing portion 22. In other words, the opening area of each the connection portion 23 (the area of the opening that is vertical in the direction in which the connection portion 23 extends from the main body portion 21) is smaller than the opening area of each the housing portion 22 (the area of the opening that is vertical in the direction in which the feeder extends). By reducing the diameter of each the connection portion 23, for example, the plant T can be easily wound around the strut 2, and the cord 24 or the like for fixing the plant T to the strut 2 can be easily entangled with each the connection portion 23.

Thus, in the an interior 2a of strut 2, the interior 22a of the hollow the housing portion 22 communicate with the interior 21a of the hollow the main body portion 21 via the connection portion 23. As a result, when the pump 7 described later air of the interior 21a of the main body portion 21, a flow path of air flowing from each the housing portion 22 toward the main body portion 21 is formed in the interior 2a of the strut 2.

The plurality of the introduction port 3 communicate between the interior 2a of the strut 2 and the external. In this embodiment, each the introduction port 3 is formed in each the housing portion 22. Each the introduction port 3 is, for example, an opening that communicates between the housing portion 22 and the external. As an example, each the introduction port 3 is positioned on an opposite portion of the housing portion 22 from the main body portion 21. The introduction port 3 includes a tapered surface 31. The tapered surface 31 expands from the interior 2a of the strut 2 (more specifically, the interior 22a in the housing portion 22) to external. In other words, at least a portion of the introduction port 3 is a countersink. The introduction port 3 can take more air in the periphery of the plant T in the interior 2a of the strut 2 by including the tapered surface 31.

The plurality of the odor sensor 4 are provided in the strut 2. In this embodiment, each the odor sensor 4 is housed in the interior 22a of each the housing portion 22, as shown in FIG. 2. As an example, each the odor sensor 4 is placed on the bottom surface on the interior 22a side of each the housing portion 22 so as to be positioned near each the introduction port 3. In this way, each the odor sensor 4 is placed at a position corresponding to each the introduction port 3 in the interior 2a of the strut 2.

Each the odor sensor 4 detects the smell of the plant T. More specifically, each the odor sensor 4 detects odorant contained in the plant T. The odor contained in the plant T is not limited to substances directly emitted from the plant T, but includes substances emitted from foreign matter attached to the plant T and substances emitted from foreign matter attached in the vicinity of the plant T.

The substance detected by the odor sensor 4 includes, for example, at least one of green scent substance, terpene substance, sulfur-odor substance, and mold base substance. The odor sensor 4 may also detect other substances in addition to green scent substance, terpene substance, sulfur-odor substance, and mold base substance.

The scent substance of green is a volatile material such as aldehyde, alcohol, and an ester thereof having a skeleton of six carbon atoms. Specific examples of green scent substances include hexenol (blue leaf alcohol) and cexenal (blue leaf aldehyde). The terpene substance is a substance such as an organic compound in which unsaturated hydrocarbon of (C₅H₈) n is a basic skeleton and terpene alcohol. Specifically, the terpene substance is α-pinene, β-caryophyllene, or the like. The sulfur base d substance is a sulfide compound, such as hydrogen sulfide, which emits the plant T putrid odor. Specific examples of the sulfur base d substance include dimethyl sulfide and the like. Examples of the mold odor substance include mold, trichloroanisole, geomine, and methyl isoborneol.

When the odor sensor 4 detects the green scent substance, the sever can detect the feeding damage state of the plant T. In addition, the detection of the terpene substance by the odor sensor 4 enables the servers to detect the environmental stress state of the plant T. In addition, the odor sensor 4 can detect the plant T base state by detecting a sulfur-damage substance. Further, when the odor sensor 4 detects mold odor substance, the server can detect mold generation of the plant T.

The odor sensor 4 is, for example, a sensor in which an organic film reacting with a detection substance is applied to a metal semiconductor. The odor sensor 4 organic film includes polyaniline, for example. The odor sensor 4 metal semiconductor includes, for example, tin oxide (SnO₂). However, the odor sensor 4 may be a sensor capable of detecting odor. The odor sensor 4 may be configured with multiple sensors when detecting multiple different odorants.

The plurality of the environmental sensor 5 are provided in the strut 2. Each the environmental sensor 5 is a sensor for detecting the growth environment of the plant T. Each the environmental sensor 5 detects at least one environment value of environment values of temperature, humidity, and carbon dioxide concentration of the plant T periphery. Each the environmental sensor 5 includes a thermometer when detecting temperature as an environment value, and includes a hygrometer when detecting humidity as an environment value. Each the environmental sensor 5 contains a carbon dioxide concentration meter if it detects carbon dioxide as an environment value. In addition, each the environmental sensor 5 may further detect an environment value other than the temperature, humidity, and carbon dioxide concentration of the plant T periphery. Each the environmental sensor 5 is attached, for example, to the outer wall surface of each the housing portion 22. The position of each the environmental sensor 5 is not limited as long as the growth environment of the plant T can be detected.

In the present embodiment, the plurality of the housing portion 22, the plurality of the introduction port 3, the plurality of the odor sensor 4, and the plurality of the environmental sensor 5 are arranged at equal intervals in the height direction of the main body portion 21.

As shown in FIG. 2, the gas pipe 6 has a hollow tubular shape and is disposed between the end of the main body portion 21 on the culture medium B side and the pump 7. The gas pipe 6 is connected to each of the end on the culture medium B side of the main body portion 21 and the pump 7. The pump 7 is connected to the strut 2 via the gas pipe 6. In particular, the pump 7 communicates with the interior 21a of the main body portion 21 via the gas pipe 6. The pump 7 is positioned, for example, adjacent to the main body portion 21 of the strut 2. The pump 7 may be placed next to the culture medium B or may be placed above the culture medium B. In FIG. 1, the pump 7 is not shown.

The pump 7 is electrically connected to a device (not shown) for power supply such as a battery and an external power source, and is driven by power supplied from the device. By driving, the pump 7 inhales air in the interior of the gas pipe 6, air in the interior 21a of the main body portion 21, and air in the interior 22a of each the housing portion 22. That is, the pump 7 inhales outside air from each the introduction port 3 formed in each the housing portion 22 into the interior 2a of the strut 2.

[Operation of Measurement Module] Next, an example of the operation of the measurement module 1 will be described. First, in the culture medium B in which the plant T is planted, the main body portion 21 of the strut 2 is erected so as to be adjacent to the stalk of the plant T. The plant T is then supported on the strut 2. To be specific, the cord 24 or the like ties the plant T to the main body portion 21 of the strut 2, a plurality of the connection portion 23, and the like. This induces the plant T to grow along the strut 2. The end on the culture medium B side of the main body portion 21 is connected to the pump 7 via the gas pipe 6. Each the odor sensor 4 and each the environmental sensor 5 are communicatively connected to the servers, and the pump 7 is electrically connected to a device for supplying power.

Subsequently, as the pump 7 actuates, the air flow of the interior 2a of the strut 2 is controlled. In particular, the pump 7 inhales air in the interior of the gas pipe 6, air in the interior 21a of the main body portion 21, and air in the interior 22a of each the housing portion 22. Thus, outside air is sucked into the interior 22a of each the housing portion 22 from each the introduction port 3 formed in each the housing portion 22. As described above, while the pump 7 is driven, in the measurement module 1, the outside air of the periphery of the plant T continues to be introduced into the interior 2a of the strut 2. In other words, while the pump 7 is driving, the plant T odor is likely to touch each the odor sensor 4. In this state, each the odor sensor 4 detects the smell of the plant T, and each the environmental sensor 5 detects at least one environment value. Data detected by each the odor sensor 4 and data detected by each the environmental sensor 5 are transmitted to the servers.

By the operation of the measurement module 1 described above, the state of the plant T can be grasped based on the data detected by each the odor sensor 4 while the data of at least one environment value is added. The servers may store the arrangement position of each the odor sensor 4 and associate the arrangement position of each the odor sensor 4 with the data detected by each the odor sensor 4. By associating the arrangement position of each the odor sensor 4 with the detected data, the servers can grasp and manage the state of the plant T in the height direction.

If each the housing portion 22 is detachably attached to the main body portion 21, the mounting position of each the housing portion 22 may optionally be changed by measurer in line with the plant T growth. The measurer can place each the odor sensor 4 at a desired location, for example, at a location where the plant T has a leaf body Ta.

[Summary of Embodiment] In the measurement module 1, a hollow the strut 2 is erected in the culture medium B to support the plant T. In the interior 2a of this the strut 2, each the odor sensor 4 is placed at a position corresponding to each the introduction port 3 communicating between the interior 2a of the strut 2 and external. The pump 7 connected to the strut 2 then draws outside air from each the introduction port 3 into the interior 2a in the strut 2. The plant T is supported on the strut 2, and the odorant emitted from the plant T is taken into the interior 2a of the strut 2 through each the introduction port 3 by suction in the pump 7 and detected by each the odor sensor 4. The odor emitted from the plant T varies depending on the state of the plant T. In this the measurement module 1, since the odor is detected by the odor sensor 4, there is no need to prepare in advance to determine the measurement site in detail and apply light to the measurement site as compared with the case where the state of the plant T is detected using light. Therefore, the measurement module 1 can continuously detect the state of the plant T even if the position of the measurement site is shifted due to growth of the plant T or application of an external force such as wind. This the measurement module 1 can be configured with a smaller number of components as compared with the case where piping and the strut 2 are separately provided by using the flow path that guides the odorant to the odor sensor 4 also the strut 2 that supports the plant T. Therefore, according to this the measurement module 1, even when the height of the plant T is high, the state of the plant T can be continuously detected with a simple configuration.

The measurement module 1 detects odorant by the odor sensor 4. Thus, the measurement module 1 can obtain data that more directly indicates the plant T 's biological information as compared to visual data obtained using a device that visually monitors the plant T, such as a camera.

In the measurement module 1, since the interior 21a of the main body portion 21 communicates with a plurality of the housing portion 22, the air flow of the interior 22a of each the housing portion 22 can be controlled even if the number of the pump 7 is one. Thus, the measurement module 1 can efficiently detect the odor of the plant T.

In addition to the function as a splint of the plant T and the support function in the odor sensor 4, the strut 2 has a function of improving the detection capability of the odor sensor 4 together with the pump 7 because the interior 2a is hollow. Therefore, the measurement module 1 can give the three functions to one the strut 2.

The measurement module 1 can incorporate odorant into each the housing portion 22 and increase the concentration of odorant in the atmosphere in each the housing portion 22 's the interior 22a. Therefore, the measurement module 1 can more accurately detect the state of the plant T.

Since each the odor sensor 4 is arranged at a position corresponding to each the introduction port 3, the measurement module 1 can detect a position corresponding to each. the plant T Therefore, the measurement module 1 can more accurately detect the state of the plant T.

Since a plurality of the introduction port 3 and a plurality of the odor sensor 4 are arranged at equal intervals in the height direction of the strut 2, the measurement module 1 can detect odors at a plurality of places from the lower side to the upper side in the plant T. Therefore, the measurement module 1 can more accurately detect the state of the plant T even if the plant T has a high height.

In general, the plant T dies from the leaf body Ta at a low position in the height direction of the plant T. Further, the leaf body Ta at a high position in the height direction of the plant T is a new leaf body. Under such a premise, for example, when the odor of the leaf body Ta in a high place is different from the odor of the leaf body Ta in other places, it is suggested that the plant T is affected by disease, feeding damage, and the like. In this regard, since the measurement module 1 can detect odors at a plurality of locations from the lower side to the upper side in the plant T, even if the health state of the plant T having a high height deteriorates, the scent can be detected early.

[Modification] While various exemplary embodiments have been described above, various omissions, substitutions and changes may be made without being limited to the exemplary embodiments described above.

In the above-described embodiment, a plurality of the introduction port 3, a plurality of the housing portion 22, a plurality of the odor sensor 4, and a plurality of the environmental sensor 5 which are arranged at equal intervals in the height direction of the strut 2 have been described. However, each of the plurality of the introduction port 3, the plurality of the housing portion 22, the plurality of the odor sensor 4, and the plurality of the environmental sensor 5 may not be arranged at equal intervals. Also, each of the number of the introduction port 3, the number of the housing portion 22, the number of the odor sensor 4, and the number of the environmental sensor 5 may be one. In this case, one the housing portion 22 may be attached to the main body portion 21, one the odor sensor 4 housed in the housing portion 22 may be placed at a position corresponding to one the introduction port 3, and one the environmental sensor 5 may be placed on the outer wall surface of the housing portion 22.

In the above embodiments, each the environmental sensor 5 was attached to the outer wall of each the housing portion 22. However, the position of each the environmental sensor 5 is not limited. Each the environmental sensor 5 may be located, for example, in the interior 22a of each the housing portion 22. In this case, since each the environmental sensor 5 detects the environment value in an environment in which the air flow is controlled by the pump 7, the measurement module 1 can reduce the variation of the environment value.

The strut 2 may not have a plurality of the connection portion 23. In that case, each the housing portion 22 may be directly connected to the main body portion 21. Each the introduction port 3 may be free of the tapered surface 31. The measurement module 1 may not include the gas pipe 6. In that case, the main body portion 21 may be directly connected to the pump 7. The measurement module 1 may not include the environmental sensor 5.

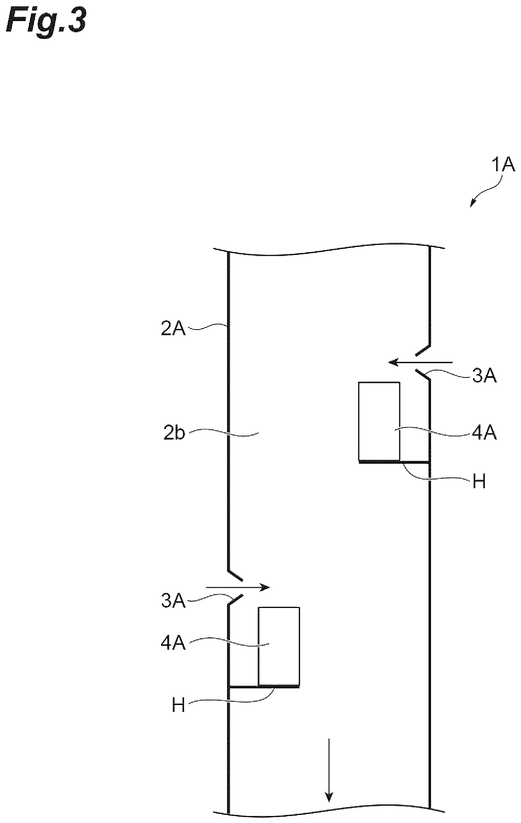

Aspects of the according to the present disclosure are not limited to the embodiments described above. FIG. 3 is a configuration diagram schematically showing an interior of a measurement module 1A according to a modification. In the example shown in FIG. 3, a strut 2A has a hollow columnar shape, and the plurality of a introduction port 3A are provided on the side surface of the strut 2A. A plurality of a odor sensor 4A are arranged in a interior 2b of the strut 2A. In particular, each the odor sensor 4A is located at a position corresponding to each the introduction port 3A in the interior 2b of the strut 2A. As an example, each the odor sensor 4A is fixed in a plate H extending from the inner wall of the strut 2A in a direction vertical to the height direction of the strut 2A. However, the fixing method of each the odor sensor 4A is not limited. According to this modified example, it is not necessary to provide a space for arranging the odor sensor 4A in the external of the strut 2A. Therefore, the measurement module 1A can save space. Also, the measurement module 1A can stabilize the center of gravity of the measurement module 1A.

## Claims

1. A measurement module (1, 1A) comprising:
a hollow strut (2, 2A) erected to support a plant (T);
at least one introduction port (3, 3A) communicating an interior (2a, 2b) and an exterior of the strut (2, 2A); and
at least one odor sensor (4, 4A) disposed at a position corresponding to the at least one introduction port (3, 3A) in the interior (2a, 2b) of the strut (2, 2A) and configured to detect an odor of the plant (T); and
a pump (7) connected to the strut (2, 2A) and configured to suck outside air from the introduction port (3, 3A) into the interior (2a, 2b) of the strut (2, 2A).

2. The measurement module (1) according to claim 1,
wherein the strut (2) comprises:
a hollow-columnar main body portion (21); and
at least one hollow box-shaped housing portion (22) in communication with an interior (21a) of the main body portion (21), and
the at least one introduction port (3) communicates between the at least one housing portion (22) and an external, and
the at least one odor sensor (4) is disposed interiorly of the at least one housing portion (22).

3. The measurement module (1) according to claim 2, wherein a connection portion between each housing portion (22) and the main body portion (21) is smaller in diameter than each housing portion (22).

4. The measurement module (1) according to claim 2 or 3, wherein the at least one housing portion (22) is detachably attached to the main body portion (21).

5. The measurement module (1, 1A) according to claim 1, wherein the strut (2, 2A) has a hollow columnar shape, and the at least one introduction port (3, 3A) is provided on a side surface of the strut (2, 2A).

6. The measurement module (1, 1A) according to any one of claims 1 to 5, wherein the introduction port (3, 3A) includes a tapered surface (31) that expands from an interior of the strut (2, 2A) toward an external.

7. The measurement module (1, 1A) according to any one of claims 1 to 6, further comprising an environmental sensor (5) configured to detect at least one of temperature, humidity, and carbon dioxide concentration of a periphery.

8. The measurement module (1, 1A) according to any one of claims 1 to 7, wherein the at least one introduction port (3, 3A) is a plurality of introduction ports, and the at least one odor sensor (4) is a plurality of odor sensors, and each of the plurality of odor sensors is disposed at a position corresponding to each of the plurality of introduction ports.

9. The measurement module (1, 1A) according to claim 8, wherein the plurality of introduction ports and the plurality of odor sensors are arranged at equal intervals in a height direction of the strut (2, 2A).

## Patentansprüche

1. Messmodul (1, 1A), umfassend:
eine hohle Strebe (2, 2A), die zum Stützen einer Pflanze (T) aufgestellt ist;
mindestens eine Einführungsöffnung (3, 3A), die ein Inneres (2a, 2b) und ein Äußeres der Strebe (2, 2A) verbindet; und
mindestens einen Geruchssensor (4, 4A), der an einer Position angeordnet ist, die der mindestens einen Einführungsöffnung (3, 3A) im Inneren (2a, 2b) der Strebe (2, 2A) entspricht, und der so konfiguriert ist, dass er einen Geruch der Pflanze (T) erfasst; und
eine Pumpe (7), die mit der Strebe (2, 2A) verbunden und so konfiguriert ist, dass sie Außenluft von der Einführungsöffnung (3, 3A) in das Innere (2a, 2b) der Strebe (2, 2A) ansaugt.

2. Messmodul (1) nach Anspruch 1,
wobei die Strebe (2) Folgendes umfasst:
einen hohlsäulenförmigen Hauptkörperabschnitt (21); und
mindestens einem hohlen, kastenförmigen Gehäuseabschnitt (22), der mit einem Innenraum (21a) des Hauptkörperabschnitts (21) in Verbindung steht, und
die mindestens eine Einführungsöffnung (3) eine Verbindung zwischen dem mindestens einen Gehäuseabschnitt (22) und einem Äußeren herstellt, und
der mindestens eine Geruchssensor (4) im Inneren des mindestens einen Gehäuseabschnitts (22) angeordnet ist.

3. Messmodul (1) nach Anspruch 2, wobei ein Verbindungsabschnitt zwischen jedem Gehäuseabschnitt (22) und dem Hauptkörperabschnitt (21) im Durchmesser kleiner ist als jeder Gehäuseabschnitt (22).

4. Messmodul (1) nach Anspruch 2 oder 3, wobei der mindestens eine Gehäuseabschnitt (22) abnehmbar an dem Hauptkörperabschnitt (21) befestigt ist.

5. Messmodul (1, 1A) nach Anspruch 1, wobei die Strebe (2, 2A) eine hohle Säulenform aufweist und die mindestens eine Einführungsöffnung (3, 3A) an einer Seitenfläche der Strebe (2, 2A) bereitgestellt ist.

6. Messmodul (1, 1A) nach einem der Ansprüche 1 bis 5, wobei die Einführungsöffnung (3, 3A) eine sich verjüngende Oberfläche (31) einschließt, die sich von einem Inneren der Strebe (2, 2A) zu einem Äußeren hin erstreckt.

7. Messmodul (1, 1A) nach einem der Ansprüche 1 bis 6, weiter umfassend einen Umgebungssensor (5), der so konfiguriert ist, dass er mindestens eines von Temperatur, Feuchtigkeit und Kohlendioxidkonzentration in der Umgebung erfasst.

8. Messmodul (1, 1A) nach einem der Ansprüche 1 bis 7, wobei die mindestens eine Einführungsöffnung (3, 3A) eine Vielzahl von Einführungsöffnungen ist, und der mindestens eine Geruchssensor (4) eine Vielzahl von Geruchssensoren ist, und jeder der Vielzahl von Geruchssensoren an einer Position angeordnet ist, die jeder der Vielzahl von Einführungsöffnungen entspricht.

9. Messmodul (1, 1A) nach Anspruch 8, wobei die Vielzahl der Einführungsöffnungen und die Vielzahl der Geruchssensoren in gleichen Abständen in einer Höhenrichtung der Strebe (2, 2A) angeordnet sind.

## Revendications

1. Module de mesure (1, 1A) comprenant :
un montant creux (2, 2A) érigé pour soutenir une plante (T) ;
au moins un orifice d'introduction (3, 3A) communiquant avec un intérieur (2a, 2b) et un extérieur du montant (2, 2A) ; et
au moins un capteur d'odeur (4, 4A) disposé à une position correspondant à l'au moins un orifice d'introduction (3, 3A) à l'intérieur (2a, 2b) du montant (2, 2A) et configuré pour détecter une odeur de la plante (T) ; et
une pompe (7) reliée au montant (2, 2A) et configurée pour aspirer un air extérieur à partir de l'orifice d'introduction (3, 3A) à l'intérieur (2a, 2b) du montant (2, 2A).

2. Module de mesure (1) selon la revendication 1,
dans lequel le montant (2) comprend :
une partie de corps principal (21) en forme de colonne creuse ; et
au moins une partie de boîtier (22) en forme de boîte creuse en communication avec un intérieur (21a) de la partie de corps principal (21), et
l'au moins un orifice d'introduction (3) communique entre l'au moins une partie de boîtier (22) et un extérieur, et
l'au moins un capteur d'odeur (4) est disposé à l'intérieur de l'au moins une partie de boîtier (22).

3. Module de mesure (1) selon la revendication 2, dans lequel une partie de connexion entre chaque partie de boîtier (22) et la partie de corps principal (21) présente un diamètre inférieur à celui de chaque partie de boîtier (22).

4. Module de mesure (1) selon la revendication 2 ou la revendication 3, dans lequel l'au moins une partie de boîtier (22) est fixée de manière amovible à la partie de corps principal (21).

5. Module de mesure (1, 1A) selon la revendication 1, dans lequel le montant (2, 2A) présente une forme de colonne creuse, et l'au moins un orifice d'introduction (3, 3A) est prévu sur une surface latérale du montant (2, 2A).

6. Module de mesure (1, 1A) selon lune quelconque des revendications 1 à 5, dans lequel l'orifice d'introduction (3, 3A) inclut une surface conique (31) qui s'étend depuis un intérieur du montant (2, 2A) vers un extérieur.

7. Module de mesure (1, 1A) selon l'une quelconque des revendications 1 à 6, comprenant en outre un capteur environnemental (5) configuré pour détecter au moins un des éléments suivants : température, humidité et concentration en dioxyde de carbone d'une périphérie.

8. Module de mesure (1, 1A) selon l'une quelconque des revendications 1 à 7, dans lequel l'au moins un orifice d'introduction (3, 3A) est une pluralité d'orifices d'introduction, et l'au moins un capteur d'odeur (4) est une pluralité de capteurs d'odeur, et chacun de la pluralité de capteurs d'odeur est disposé à une position correspondant à chacun de la pluralité d'orifices d'introduction.

9. Module de mesure (1, 1A) selon la revendication 8, dans lequel la pluralité d'orifices d'introduction et la pluralité de capteurs d'odeur sont disposés à intervalles égaux dans une direction de hauteur du montant (2, 2A).
